# EUROPÄISCHE PATENTSCHRIFT

(11) **EP 2 567 677 B1**
(45) Veröffentlichungstag und Bekanntmachung des Hinweises auf die Patenterteilung: **18.02.2015**
(21) Anmeldenummer: 12183738.9
(22) Anmeldetag: 10.09.2012
(51) Int. Cl.: A61F 2/42, A61F 2/30

(54) **Subtalar-Implantat**
Subtalar implant
Implant sous-astragalien

(30) Priorität: 08.09.2011 DE 102011053417
(43) Veröffentlichungstag der Anmeldung: 13.03.2013
(73) Patentinhaber: Zimmer GmbH, 8404 Winterthur (CH)
(72) Erfinder: Pech, Uwe, 78532 Tuttlingen (DE); Kotuljac, Vladko, 72355 Schömberg (DE)
(74) Vertreter: Manitz, Finsterwald & Partner GbR

(56) Entgegenhaltungen:
- EP-A1- 0 560 249
- EP-A1- 1 728 491
- US-A1- 2002 040 243
- US-A1- 2005 251 264
- US-A1- 2011 071 579

## Beschreibung

Die Erfindung betrifft ein Subtalar-Implantat gemäß dem Oberbegriff des Anspruchs 1 zur Stabilisierung des Subtalargelenks im menschlichen Fuß, mit einer sich zwischen einem vorderen Ende und einem hinteren Ende erstreckenden (gedachten) Längsachse, mit einem seitlich der Längsachse angeordneten ersten Ankerabschnitt und einem seitlich der Längsachse angeordneten zweiten Ankerabschnitt.

Zur Behandlung von Plattfüßen ist es bekannt, bei der Operation zur Stabilisierung des Subtalargelenks des menschlichen Fußes zwischen Calcaneus und Talus ein Subtalar-Implantat einzudrehen. Aus der Praxis bekannte Subtalar-Implantate sind einstückig ausgebildet, weisen einen Durchgangskanal zur Aufnahme eines Kirschnerdrahtes auf und haben eine fixe konische Hüllkontur, die mantelseitig mit einem Gewinde strukturiert ist, um das Implantat im Calcaneus sowie im Talus verankern zu können. Subtalar-Implantate gibt es in verschiedenen Größen, von denen der Operateur während der Operation ein geeignetes (passendes) Subtalar-Implantat auswählen muss, wobei es vorkommen kann, dass das ausgewählte Implantat nicht optimal passt, so dass dieses wieder entfernt und durch ein geeigneteres Subtalar-Implantat ersetzt werden muss.

Aus der US 2011/0071579 A1 ist eine biokompatible Spreizvorrichtung bekannt. Diese umfasst in dem Ausführungsbeispiel gemäß den Fig. 15 und 16 eine Schraube und eine auf der Schraube verdrehbare Mutter, wobei durch Betätigen der Schraube die Mutter in Richtung Schraubenkopf verstellt wird und dadurch Ankerabschnitte, die an ihrem Außenumfang glatt sind, nach radial außen verstellt werden. Der Antrieb der Schraube befindet sich auf der von der Öffnungsseite der Spreizabschnitte abgewandten Axialseite.

Die bekannte Expansionsvorrichtung ist mehrteilig ausgebildet, wobei die Mutter auch vollständig von der Schraube abgedreht werden kann, welche wiederum aus ihrer Aufnahme entfernbar ist.

Ein weiterer Spreizmechanismus ist in der US 2002/0040243 A1 beschrieben. Hier wird eine Madenschraube in einer Innengewindeöffnung verstellt und verschiebt dadurch ein Spreizelement, welches Ankerabschnitte nach radial außen aufweitet.

Die US 2005/0177243 A1 zeigt ein einteiliges Subtalar-Implantat, welches an einem Kirschnerdraht geführt ist und welches ausschließlich durch Verschrauben an die entsprechende Körperstelle bringbar ist.

Aus der DE 693 12 532 T2 ist ein aufspreizbares Subtalar-Implantat bekannt, wobei hierzu eine Spreizschraube in einen entsprechenden Aufspreizkörper eingedreht wird. Ein Herausdrehen der Schraube ist möglich.

Aus der DE 40 00 112 A1 ist ein mehrteiliger Spreizkörper bekannt.

Ausgehend von dem vorgenannten Stand der Technik liegt der Erfindung die Aufgabe zugrunde, ein die Operation vereinfachendes (und vorzugsweise beschleunigendes), verbessertes Subtalar-Implantat zur Stabilisierung des Subtalargelenks im menschlichen Fuß, insbesondere zur Behandlung von Plattfüßen anzugeben. Die Gefahr von Operationsfehlern soll reduziert werden, insbesondere soll die Operationssicherheit optimiert werden. Zudem soll die Notwendigkeit zur Bevorratung einer Vielzahl von unterschiedlich großen Implantaten entfallen.

Diese Aufgabe wird mit einem Subtalar-Implantat mit den Merkmalen des Anspruchs 1 gelöst, bei einem gattungsgemäßen Subtalar-Implantat insbesondere dadurch, dass das Subtalar-Implantat Spreizmittel zum Ausspreizen des Implantates durch Relativverstellen des ersten und des zweiten Ankerabschnitts winklig zur Längsachse vorgesehen sind.

Vorteilhafte Weiterbildungen der Erfindung sind in den Unteransprüchen angegeben. In den Rahmen der Erfindung fallen sämtliche Kombinationen aus zumindest zwei von in der Beschreibung, den Ansprüchen und/oder den Figuren offenbarten Merkmalen.

Der Erfindung liegt der Gedanke zugrunde, das Subtalar-Implantat aufweitbar, d.h. aufspreizbar auszugestalten, um auf diese Weise die Radial-Erstreckung des Subtalar-Implantates, insbesondere von dessen Ankerabschnitten bezogen auf die Längsachse während der Operation einstellen zu können. Dem Subtalar-Implantat sind hierzu integrale Spreizmittel zugeordnet, um einen zwischen den mindestens zwei Ankerabschnitten aufgespannten Winkel (Spreizwinkel) verstellen zu können. Durch die erfindungsgemäße Ausgestaltung des Subtalar-Implantates kann somit auf die Auswahl aus einer Vielzahl von starren bzw. unverstellbaren Subtalar-Implantaten, in der Praxis üblicherweise acht, verzichtet werden. Zumindest kann für den Fall des Vorsehens von mehreren Subtalar-Implantaten mit unterschiedlichen Spreizweiten bzw. -bereichen die Auswahlmöglichkeit stark eingeschränkt werden. Insgesamt kann durch das Subtalar-Implantat gemäß der Erfindung die Operationszeit verkürzt und die Gefahr einer Falschauswahl aufgrund der Möglichkeit zur Anpassung der Radialerstreckung sowie der geringen Anzahl unterschiedlicher, sich in den maximalen und/oder minimalen Spreizweiten unterscheidenden Implantaten zumindest deutlich reduziert, insbesondere im Falle des Vorsehens nur eines Implantates völlig eliminiert werden.

Das erfindungsgemäße Subtalar-Implantat eignet sich zur Behandlung von Plattfüßen, insbesondere bei Kindern.

Die Spreizmittel umfassen einen Spindeltrieb, mit welchem die Ankerabschnitte aufgespreizt werden können, wobei es besonders bevorzugt ist, wenn sich eine Spindel des Spindeltriebs in Richtung der Längsachse des Subtalar-Implantates erstreckt.

Erfindungsgemäß ist vorgesehen, dass die Spindel des Spindeltriebes drehbar gelagert ist in einem Basisabschnitt des Implantates. Die Spindel umfasst bevorzugt rückwärtig, insbesondere im Bereich ihres, der Implantierrichtung entgegengesetzten freien Endes, einen Antrieb, beispielsweise einen Inbus- oder Torxantrieb, mit welchem die Spindel relativ zu den Ankerabschnitten verdrehbar ist, wobei durch Verdrehen der Spindel ein mit der Spindel in Eingriff stehender Spreizreiter entlang der Längsachse der Spindel verstellbar ist. Bevorzugt greift der Spreizreiter mit einem Innengewinde in ein äußeres Spindelgewinde ein. Im Falle des Vorsehens einer verdrehbaren Spindel stützt sich der Spreizreiter bevorzugt drehfest am Innenumfang der Ankerabschnitte ab.

Im Hinblick auf die Ausgestaltung einer gut sterilisierbaren und einfach aufgebauten und für die Operationssicherheit optimalen Axialsicherung der Spindel ist erfindungsgemäß vorgesehen, dass diese unverlierbar am Implantat gehalten (gesichert) ist, in dem sie mit einem verdickten Abschnitt in einer Kavität des Subtalar-Implantates aufgenommen ist, wobei die Kavität zumindest abschnittsweise von einem fakultativ vorgesehenen Basisabschnitt gebildet ist. Erfindungsgemäß ist vorgesehen, dass ein vorderer Rand des Basisabschnittes nach axialem Einschieben der Spindel umgebördelt wird und diese somit fixiert.

Die axiale Sicherung bildet in diesem Fall gleichzeitig ein Gleitlager zum drehbaren Lagern der Spindel. Durch die axiale Sicherung wird ein Verlieren der Spindel während der Operation sicher verhindert.

Ganz besonders bevorzugt ist es, wenn die Ankerabschnitte, mit denen das Subtalar-Implantat im Sinus-Tarsi-Tunnel festlegbar bzw. in Knochen, insbesondere im Calcaneus sowie im Talus verankerbar ist, Bestandteil einer (gedachten) konischen Hüllkontur des Subtalar-Implantates sind, wobei in diesem Fall bevorzugt der einstellbare Spreizwinkel dem Konuswinkel dieser Hüllkontur entspricht. Dieser kann je nach Ausgestaltung der Spreizmittel in Stufen oder bevorzugt stufenlos einstellbar sein.

Besonders zweckmäßig ist eine Ausführungsform, bei der der Antrieb der Spindel, welcher vorzugsweise stirnseitig in der Spindel beispielsweise als Sechskantantrieb oder Torxantrieb ausgebildet sein kann auf einer Axialseite des Implantates angeordnet ist, an der die Ankerabschnitte im gespreizten Zustand maximal beabstandet sind. Anders ausgedrückt weist der Antrieb also bevorzugt entgegen der axialen Einbringrichtung des Implantates bzw. befindet sich auf der (bevorzugt entgegen der Einbringungsrichtung orientierten) Öffnungsseite des Implantates, da das Subtalar-Implantat hierdurch besonders für die Implantation im Sinus-Tarsi-Tunnel geeignet ist.

Erfindungsgemäß ist vorgesehen, die Spindel unverlierbar zu sichern. Weiterbildungsgemäß sind sämtliche Einzel- bzw. Bauteile des Subtalar-Implantates unverlierbar angeordnet, insbesondere der Spreizreiter. Hierzu bietet es sich in Weiterbildung der Erfindung an, wenn zumindest einer der Ankerabschnitte, insbesondere sämtliche, ganz besonders beide Ankerabschnitte den Spreizreiter zur axialen Sicherung in radialer Richtung bezogen auf die Axialerstreckung der Spindel übergreift/übergreifen.

Dabei muss ein Übergreifen nicht auf der gesamten Axialerstreckung der Spindel realisiert sein, sondern zumindest bei zusammengefahrenen Ankerabschnitten, um ein Abdrehen des Spreizreiters von der Spindel durch Betätigung der Spindel sicher zu vermeiden. Bevorzugt weist hierzu der mindestens eine Ankerabschnitt eine entsprechende Aufnahmetasche und/oder eine radiale Erweiterung für den Spreizreiter auf.

Ganz besonders zweckmäßig ist es, wenn der Spreizreiter drehfest angeordnet ist, so dass dieser bei Verdrehen der Spindel relativ zu dieser entlang von deren Längserstreckung wandern kann. Besonders zweckmäßig ist es dabei, wenn sich der Spreizreiter an den Ankerabschnitten abstützt. Noch weiter bevorzugt ist es dabei, wenn der Spreizreiter eine in Draufsicht rechteckige Hüllkontur aufweist, wobei es noch weiter bevorzugt ist, wenn der Spreizreiter insgesamt eine quaderförmige Hüllkontur aufweist.

Zum erleichterten Implantieren des vorgeschlagenen Subtalar-Implantates hat es sich als vorteilhaft herausgestellt, wenn an dem Spreizreiter ein Antrieb, insbesondere in Form einer Verdrehsicherung, insbesondere in Form von seitlichen Aussparungen vorgesehen ist, um mit entsprechenden Fortsätzen eines Implantationswerkzeuges zusammenwirken zu können, so dass ein unbeabsichtigtes Verdrehen des Subtalar-Implantates bei einem Verdrehen des Spindelantriebs sicher vermieden wird. Gleichzeitig kann über den Antrieb ein Drehmoment aufgebracht werden, um das Subtalar-Implantat, insbesondere im Falle des Vorsehens eines Außengewindes an den Ankerabschnitten zwischen zwei Knochen eindrehen zu können und/oder um das Implantat wieder entfernen zu können, insbesondere durch Beaufschlagung mit einer Zugkraft und/oder einem Drehmoment.

Jedenfalls soll die Verdrehsicherung eine Relativverdrehbewegung zwischen Subtalar-Implantat bzw. zwischen den Ankerabschnitten des Subtalar-Implantates und dem Implantationswerkzeug sicher vermeiden.

Die mindestens zwei Ankerabschnitte sind vorzugsweise zumindest in einem aufgespreizten Zustand in Umfangsrichtung voneinander beabstandet. Besonders zweckmäßig ist es, wenn sich die Ankerabschnitte jeweils nur über einen Teilumfangsabschnitt des Subtalar-Implantates erstrecken. Als besonders zweckmäßig hat es sich herausgestellt, wenn, insbesondere im Falle des Vorsehens von ausschließlich zwei Ankerabschnitten, sich jeder Ankerabschnitt über einen Umfangswinkel zwischen etwa 60° und etwa 120° erstreckt. Besonders zweckmäßig ist es, wenn die Umfangserstreckung jedes Ankerabschnitts in etwa 90° beträgt.

Im Falle des Vorsehens von insgesamt zwei relativ zueinander verstellbaren bzw. auseinanderspreizbaren Ankerabschnitten ist es bevorzugt, wenn diese - bezogen auf die gedachte Längsachse diametral gegenüberliegend angeordnet sind. Im Fall des Vorsehens von mehr als zwei Ankerabschnitten sind diese in Umfangsrichtung verteilt angeordnet, wobei es besonders bevorzugt ist, wenn die Ankerabschnitte gleichmäßig in Umfangsrichtung verteilt angeordnet sind.

Um eine korrekte Positionierung des Subtalar-Implantates zwischen zwei Knochen im Fuß während der Operation zu ermöglichen, ist in Weiterbildung der Erfindung ein axialer Durchgangskanal vorgesehen, der sich zwischen dem vorderen und dem hinteren Ende des Subtalar-Implantates erstreckt, wobei der Durchgangskanal zur Aufnahme eines Kirschnerdrahtes ausgelegt und bestimmt ist.

Besonders zweckmäßig ist es im Falle einer später noch zu erläuternden Ausbildung der Spreizmittel als Spindeltrieb, wenn der Durchgangskanal eine zentrische, verdrehbar angeordnete oder alternativ drehfeste Spindel mittig durchsetzt.

Wie eingangs erläutert, dienen die mindestens zwei Ankerabschnitte dazu, das Subtalar-Implantat sicher im menschlichen Knochen zu halten, wozu die Ankerabschnitte in die Knochen eingeformt (verankert) werden müssen. Hierzu sind die Ankerabschnitte an ihrer Außenfläche mit einer Oberflächenstruktur versehen, um das Subtalar-Implantat, beispielsweise durch Einschlagen, Einschrauben und/oder durch den Spreizvorgang ein stückweit in den Knochen einformen zu können. Besonders zweckmäßig ist es, wenn auf den Ankerabschnitten ein Außengewinde vorgesehen ist, wobei sich dieses Außengewinde dann über die Ankerabschnitte erstreckt bzw. die Teilstrukturierungen Teil eines gemeinsamen Außengewindes bilden. Alternativ ist es beispielsweise denkbar eine Rillenstruktur vorzusehen, wobei derart strukturierte Ankerabschnitte bevorzugt durch Einschlagen und/oder Aufspreizen in den Knochen eingegraben werden. Besonders zweckmäßig ist es, wenn die Oberflächenstruktur wieder hakenartig ausgebildet ist, jedenfalls so, dass ein unbeabsichtigtes Verstellen entgegen einer Einbringrichtung sicher vermieden wird.

Im Hinblick auf eine gute Sterilisierbarkeit sowie einen einfachen mechanischen Aufbau hat es sich als vorteilhaft herausgestellt, wenn mindestens zwei der Ankerabschnitte, insbesondere sämtliche Ankerabschnitte zusammen mit einem diese verbindenden Verbindungsabschnitt (Basisabschnitt) einstückig, d.h. aus einem Materialstück zusammenhängend (d.h. nicht mehrteilig) gefertigt sind. Bevorzugt bildet der Basisabschnitt gleichzeitig eine Halterung für die Spreizmittel, insbesondere für die Spindel, die weiter bevorzugt an dem Basisabschnitt verdrehbar festgelegt ist.

Als besonders zweckmäßig hat es sich herausgestellt, die Ankerabschnitte federnd auszubilden und/oder anzuordnen, insbesondere derart, dass diese bestrebt sind, einer Spreizkraft entgegenzuwirken, d.h. nach radial innen zu federn. Auf diese Weise kann konstruktiv sehr einfach sichergestellt werden, dass die Ankerabschnitte bei einem entsprechenden Betätigen der Spreizmittel wieder entspreizen, um hierdurch weiterbildungsgemäß aufgrund der resultierenden Durchmesserreduzierung erleichtert entnommen werden zu können.

Um das Subtalar-Implantat langfristig im Körper verdrehsicher anzuordnen, ist in Weiterbildung der Erfindung in zumindest einem der Ankerabschnitte eine Längsnut als Rotationssicherung vorgesehen, wobei die Längsnut bevorzugt nach hinten, d.h. entgegen der Einbringrichtung geöffnet ist. In die Längsnut kann Knochenmaterial eindringen, insbesondere einwachsen und somit zur Verdrehsicherung beitragen.

Bevorzugt unterbricht die Längsnut eine bevorzugt als Außengewinde ausgebildete Oberflächenstruktur der Ankerabschnitte und bildet somit Schneidkanten, insbesondere Außengewindeschneidkanten, wodurch ein etwaig vorgesehenes Außengewinde zu einem selbstschneidenden Gewinde ausgebildet wird, um hierdurch ein erleichtertes Lösen zu Zwecken der Entfernung des Implantates zu ermöglichen.

Besonders zweckmäßig ist es, wenn das Subtalar-Implantat mindestens um ein Maß spreizbar ist, so dass dessen maximaler (veränderbarer) Durchmesser um mindestens 2mm veränderbar ist, ganz besonders bevorzugt um mehr als 3mm oder um etwa 3mm. Bevorzugt ist der (variable) maximale Durchmesser aus einem Wertebereich zwischen 5mm und 20mm, vorzugsweise zwischen 10mm und 16mm, gewählt.

Ganz besonders bevorzugt ist es, wenn mindestens zwei unterschiedliche Subtalar-Implantate in einem Implantatsortiment zusammengestellt sind, wobei ein erstes der Implantate in einem ersten Durchmesserbereich und ein zweites der Implantate in einem zweiten Durchmesserbereich variierbar sind, wobei die Durchmesserbereiche sich überschneiden können, oder alternativ nicht. So ist es beispielsweise möglich, und bevorzugt ein Implantat so auszubilden, dass dessen maximaler Durchmesser zwischen 10mm und 13mm verstellbar ist. Zusätzlich oder alternativ kann ein Implantat in einem Implantatsortiment vorhanden sein, dessen maximaler Durchmesser in einem Bereich zwischen 13mm und 16mm einzustellen ist.

Um das Subtalar-Implantat erleichtert bei einer späteren Operation wieder entfernen zu können, ist in Weiterbildung der Erfindung mit Vorteil vorgesehen, dass der maximale Außendurchmesser des Subtalar-Implantates verkleinerbar ist, insbesondere durch entsprechendes Betätigen der Spreizmittel entgegen der Verdrehrichtung beim Implantationsvorgang. Ganz besonders bevorzugt ist es, wenn die Ankerabschnitte nach radial innen federnd ausgebildet sind, damit diese automatisch, ohne mechanisch mitgenommen zu werden, wieder in Richtung ihrer Ursprungslage tendieren.

Die Erfindung führt auch auf ein System, umfassend ein nach dem Konzept der Erfindung ausgebildetes Subtalar-Implantat und ein Werkzeug zum Einbringen und/oder Entfernen eines Subtalar-Implantates. Bevorzugt ist das Werkzeug derart ausgebildet, dass es formschlüssig mit einem Antrieb oder Verdrehsicherungsmitteln des Spreizreiters zusammenwirkt, der sich vorzugsweise in Umfangsrichtung drehfest an mindestens einem der Ankerabschnitte abstützt.

Bevorzugt ist das Werkzeug dabei so ausgebildet, dass, wenn mit diesem der Spreizreiter gehalten ist, über einen entsprechenden, rotierbaren Betätigungsmechanismus, der mit dem Antrieb der Spindel zusammenwirkt, diese verdreht werden kann. Auch ist es möglich, dass ein Werkzeug eingesetzt wird, das nicht integral diese Verdrehmittel zum Verdrehen der Spindel aufweist, sondern mit welchem ausschließlich zum Einbringen oder Lösen des Implantates ein Formschluss mit dem Spreizreiter hergestellt werden kann, insbesondere um ein Drehmoment in dieses und damit in das gesamte Implantat einzubringen und/oder um eine Zug- oder Druckkraft auszuüben.

Besonders bevorzugt ist eine Ausführungsvariante des Subtalar-Implantates mit zentrischer Durchgangsöffnung zur Aufnahme eines Kirschnerdrahtes, der eine eignete Positionierung und auch Führung von Werkzeugen zum Betätigen des Implantates, insbesondere der Spindel erlaubt.

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:
- Fig. 1a bis Fig. 1e: unterschiedliche Ansichten eines bevorzugten Ausführungsbeispiels eines Subtalar-Implantates in einem nicht expandierten Zustand, und

- Fig.2a bis Fig. 2d: unterschiedliche Ansichten des Subtalar-Implantates gemäß den vorstehenden Figuren in einem aufgespreizten Zustand, und
- Fig.3a bis Fig.3d:: unterschiedliche Ansichten eines alternativen Subtalar-Implantates.

In den Figuren sind gleiche Elemente und Elemente mit der gleichen Funktion und den gleichen Bezugszeichen gekennzeichnet.

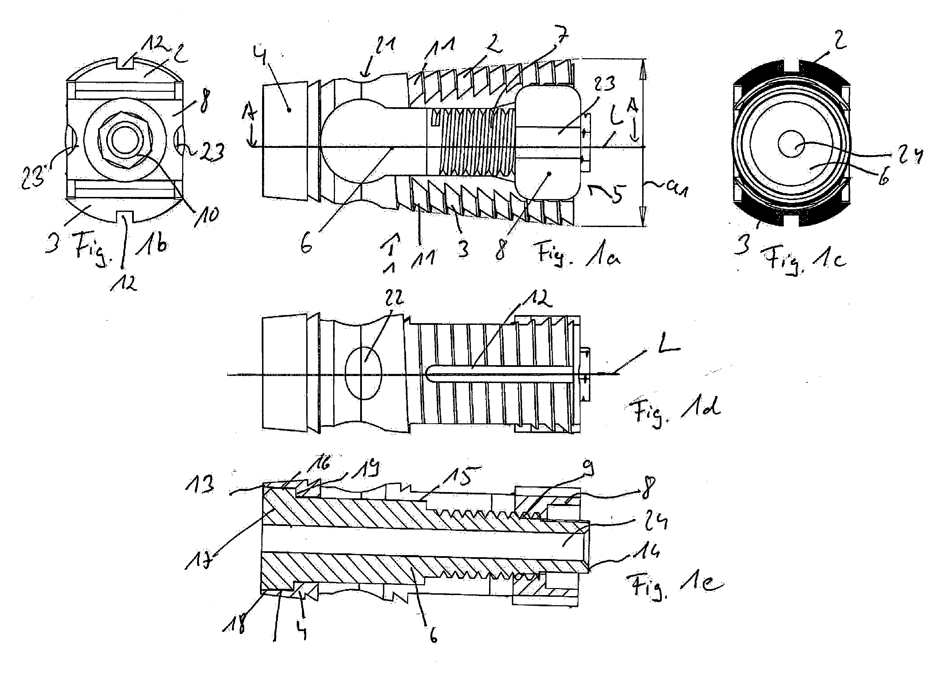

In den Fig. 1a bis 1d ist ein Subtalar-Implantat im nicht aufgespreizten Zustand dargestellt, wobei Fig. 1e eine Schnittdarstellung entlang der Schnittlinie A-A gemäß Fig. 1 a zeigt.

Das dreiteilige Subtalar-Implantat 1 umfasst einen ersten Ankerabschnitt 2, einen diametral gegenüberliegenden zweiten Ankerabschnitt 3 sowie einen einteilig mit den Ankerabschnitten 2, 3 ausgebildeten (vorderen) Basisabschnitt 4, wobei Ankerabschnitte 2, 3 und Basisabschnitt 4 eine Art Hülle oder Gehäuse bilden, welches als ein erstes der drei Bauteile des Subtalar-Implantates zu betrachten ist. Ferner umfasst das Subtalar-Implantat Spreizmittel 5, die zweiteilig ausgebildet sind und in dem konkreten Ausführungsbeispiel aus einer drehbar gelagerten Spindel 6 bestehen, die abschnittsweise mit einem Außengewinde 7 versehen ist, sowie einem Spreizreiter 8, der mit einem Innengewinde 9 in Eingriff ist mit dem Außengewinde 7 (Spindelgewinde) der Spindel 6. Die Spindel 6 ist an ihrem rückwärtigen Ende mit einem in dem gezeigten Ausführungsbeispiel lediglich beispielhaft als Außensechskant ausgebildeten Antrieb 10 versehen.

Durch Verdrehen des Antriebs 10 kann der Spreizreiter 8 relativ zu den Ankerabschnitten 2, 3 entlang einer Längsachse L wandern, und dabei die Ankerabschnitte 2, 3 relativ zueinander verstellen, die sich radial innen unmittelbar am Spreizreiter 8 abstützen.

Zu erkennen ist, dass in dem gezeigten Ausführungsbeispiel selbst in der vollständig entspreizten, d.h. zusammengefahrenen Stellung die Ankerabschnitte 2, 3 eine leicht konische Hüllkontur aufweisen, alternativ kann die Anordnung zumindest im dargestellten entspreizten Zustand auch zylindrisch konturiert sein, wobei die gezeigte Ausführungsform den Vorteil hat, dass auch in dem entspreizten Zustand ein Verankern in den betroffenen Knochen im Fuß möglich ist.

Beide Ankerabschnitte 2, 3 erstrecken sich lediglich über einen Umfangsabschnitt des Subtalar-Implantates, in dem gezeigten Ausführungsbeispiel jeweils über einen Umfangswinkel von etwa 90°. Die Ankerabschnitte 2, 3 sind an ihrem Außenumfang mit einer Oberflächenstruktur 11 (Verankerungsstruktur) versehen, wobei die Überflächenstrukturen 11 beider Ankerabschnitte 2, 3 in dem gezeigten Ausführungsbeispiel Teil eines gemeinsamen Außengewindes sind, also eine Steigung aufweisen, um zu ermöglichen, das Subtalar-Implantat 1 drehend in den Knochen zu verankern. Alternativ kann beispielsweise auch auf die Steigung verzichtet werden und eine Rillenstruktur vorgesehen werden, oder alternativ eine andere Verankerungsstruktur. Sichergestellt werden muss jedoch, dass sich die Ankerabschnitte 2, 3 in den Knochen verankern lassen.

Aus Fig. 1d ist zu entnehmen, dass in den Ankerabschnitten 2, 3 jeweils eine nach hinten offene Längsnut 12 vorgesehen ist, die im implantierten Zustand als Antrieb und/oder Verdrehsicherung dient. Alternativ können in zumindest einem der Ankerabschnitt auch mehrere, beispielsweise parallele Längsnuten zur Verbesserung der Verdrehsicherung vorgesehen werden.

Aus Fig. 1e (Längsschnittansicht) ist zu erkennen, dass die Spindel 6 von einem vorderen Ende 13 des Implantates 1 montierbar ist, in dem die Spindel 6 in Richtung einem hinteren Ende 14 des Implantates 1 in eine von dem Basisabschnitt 4 sowie den Ankerabschnitten 2, 3 begrenzten Spindelaufnahme 15 einschiebbar ist. In einem vorderen Bereich des Basisabschnittes 4 ist eine Kavität 16 gebildet, in welcher ein verdickter vorderer Abschnitt 17 (vergrößerter Durchmesserabschnitt) der Spindel 6 aufnehmbar ist. Durch Umbördeln eines vorderen Umfangsrandes 18 des Basisabschnittes 4 kann die Spindel 6 nach axial vorne gesichert werden. Nach axial hinten stützt sich der verdickte Abschnitt 17 an einer inneren Ringschulter 18 der Kavität 16 ab. Wie sich unschwer aus den Zeichnungen erkennen lässt, ist das Subtalar-Implantat 1 (aus Übersichtlichkeitsgründen) noch nicht endmontiert - es bedarf noch des Umbördelns des Umfangsrandes 18.

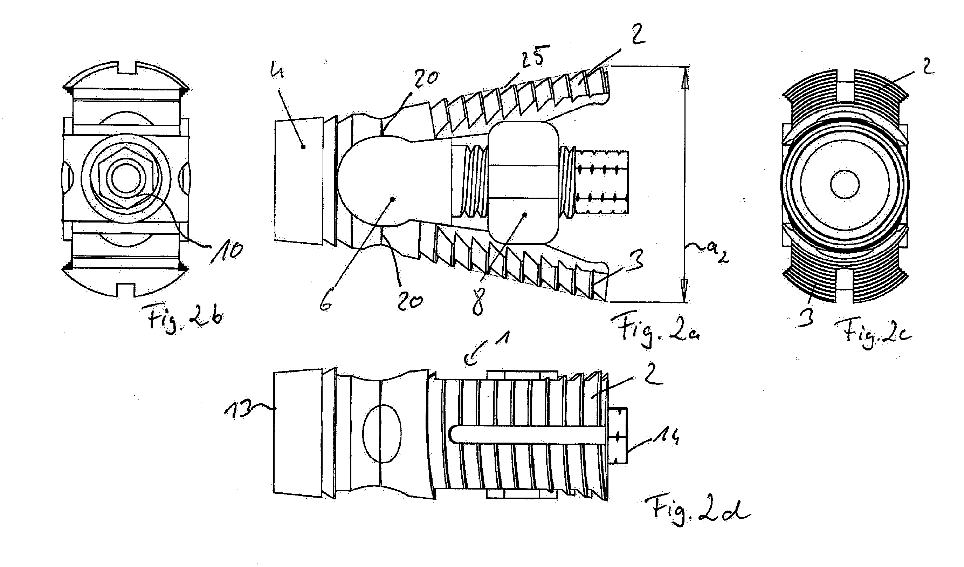

Die Ankerabschnitte 2, 3 sind federelastisch aufgebaut. Zur Verdeutlichung der Federelastizität ist in Fig. 2a ein Spalt 20 angedeutet, der in der Realität nicht vorhanden ist - hier findet keine Aufweitung statt, sondern lediglich eine elastische Verformung. Die Federkraft der Ankerabschnitte 2, 3, die bestrebt sind nach radial innen in Richtung Längsmittelachse L zu federn, kann durch eine Anpassung der Materialdicke in einem Übergangsbereich 21 zwischen den umfangsgeschlossenen Basisabschnitt 4 und den eigentlichen, oberflächenstrukturierten Ankerabschnitten 2, 3 gewählt werden, sowie durch eine Beeinflussung der Übergangsabschnittsgeometrie, beispielsweise durch die Wahl des Krümmungsradius einer seitlichen Vertiefung 22.

Aus Fig. 1a ist zu erkennen, dass an dem im Querschnitt in etwa rechteckig konturierten Spreizreiter 8 zwei aneinander gegenüberliegende seitliche, hier konkav konturierte und sich in Längsrichtung erstreckenden Aussparungen 23 vorgesehen sind, in die ein entsprechendes Implantationswerkzeug (nicht dargestellt) eingreifen kann, um ein Verdrehen des Spreizreiters 8 und damit auch der Ankerabschnitte 2, 3 relativ zu dem Implantationswerkzeug zu verhindern, insbesondere wenn mit einem Implantationswerkzeug der Antrieb 10 betätigt wird. Auch kann mit Hilfe der Aussparungen 23 ein Drehmoment mittels des Implantationswerkzeuges auf das Subtalar-Implantat 1 aufgebracht werden, um eine Verankerung in dem Knochen zu erleichtern.

Zu erkennen ist in Fig. 1b auch, dass der Spreizreiter einen gedachten, von den Ankerabschnitten 2, 3 abschnittsweise beschriebenen Kreis nach radial außen nicht überschreitet, damit der Spreizreiter 8 ein mögliches gewolltes Verdrehen des Subtalar-Implantates 1 mittels eines Implantationswerkzeug nicht behindert.

Aus Fig. 1e ist ersichtlich, dass die Spindel 6 durchsetzt ist von einem Durchgangskanal 24, der zur Aufnahme eines Kirschnerdrahtes und/oder eines Axialfortsatzes eines möglichen Implantationswerkzeuges dienen kann.

In den Fig. 2a bis 2d ist das Subtalar-Implantat 1 in einer aufgespreizten Position gezeigt. Diese wurde durch Betätigen des Antriebs 10 und damit Verdrehen der Spindel 6 eingestellt, wodurch der Spreizreiter 8 in der Folge in der Zeichnungsebene nach links, d.h. in Richtung des vorderen Endes 13 des Subtalar-Implantates 1 gewandert ist und hierdurch die Ankerabschnitte 2, 3 aufgeweitet, d.h. radial bezogen auf die Längsmittelachse L verstellt hat, wodurch sich ein Konuswinkel (Winkel zwischen zwei diametral gegenüberliegenden Mantelflächenabschnitten der konischen Hüllkontur 25 verändert) hier vergrößert hat.

Das in den Figuren gezeigte Subtalar-Implantat 1 lässt sich zwischen einem in Fig. 1a eingezeichneten Außendurchmesser a1 von 10mm verstellen bis auf einen in Fig. 2a eingezeichneten Außendurchmesser a2 von 13mm.

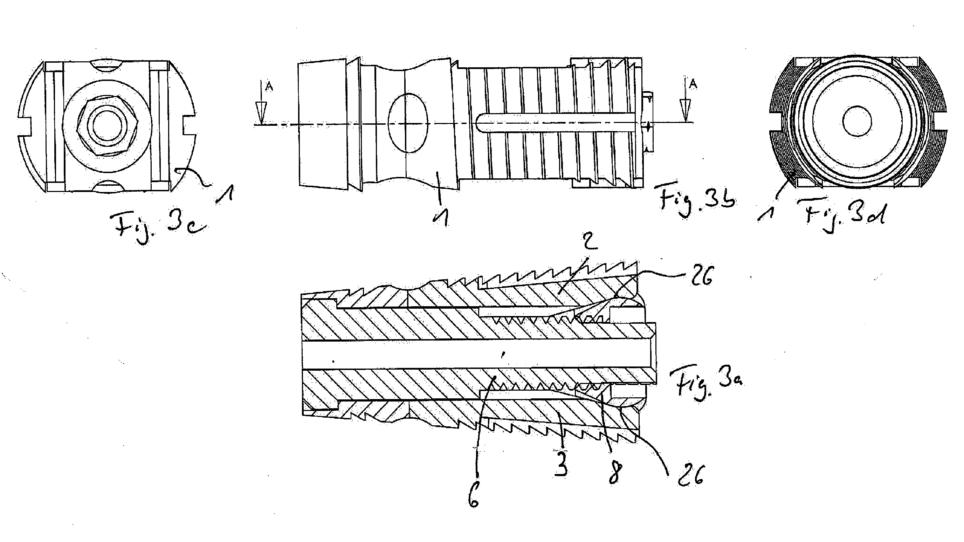

Das in den Fig. 3a bis 3d dargestellte Subtalar-Implantat entspricht im Wesentlichen dem zuvor beschriebenen Subtalar-Implantat gemäß den vorhergehenden Figuren, so dass in Bezug auf Gemeinsamkeiten auf entsprechende Offenbarungsstellen verwiesen wird. Im Nachfolgenden wird im Wesentlichen auf die Unterschiede eingegangen. Zur Vermeidung von Wiederholungen wird hinsichtlich der Gemeinsamkeiten auf vorstehende Beschreibung inklusive Figuren verwiesen.

Der Hauptunterschied besteht darin, dass der Spreizreiter 8 unverlierbar an der Spindel 6 gehalten ist. Die axiale Sicherung erfolgt über entsprechende innere Einbuchtungen 26 am Innenumfang der Ankerabschnitte 2, 3, in denen der Spreizreiter im nicht aufgespreizten Zustand aufgenommen ist. Anders ausgedrückt übergreifen die Ankerabschnitte 2, 3 den Spreizreiter 8 in radialer Richtung nach innen, so dass ein Ablösen des Spreizreiters 8 über die dargestellte Position hinaus hin zur Öffnungsseite sicher vermieden wird.

### Bezugszeichenliste

- 1: Subtalar-Implantat
- 2: erster Ankerabschnitt
- 3: zweiter Ankerabschnitt
- 4: Basisabschnitt
- 5: Spreizmittel
- 6: Spindel
- 7: Außengewinde (Spindelgewinde)
- 8: Spreizreiter
- 9: Innengewinde
- 10: Antrieb
- 11: Oberflächenstruktur
- 12: Längsnut
- 13: vorderes Ende
- 14: hinteres Ende
- 15: Spindelaufnahme
- 16: Kavität
- 17: verdickter Abschnitt der Spindel
- 18: Umfangsrand
- 19: Ringschulter
- 20: Spalt
- 21: Übergangsabschnitt
- 22: Vertiefung
- 23: Aussparung
- 24: Durchgangskanal
- 25: Hüllkontur
- 26: Einbuchtungen
- L: Längsachse
- a1: kleinster maximaler Durchmesser
- a2: größter maximaler Durchmesser

## Patentansprüche

1. Subtalar-Implantat zur Stabilisierung des Subtalargelenks im menschlichen Fuß, mit einer sich zwischen einem vorderen Ende (13) und einem hinteren Ende (14) erstreckenden Längsachse (L), mit einem seitlich der Längsachse (L) angeordneten ersten Ankerabschnitt (2) und einem seitlich der Längsachse (L) angeordneten zweiten Ankerabschnitt (3), wobei Spreizmittel (5) zum Aufspreizen des Implantates (1) durch Relativverstellen des ersten und des zweiten Ankerabschnittes (2, 3) vorgesehen sind, und wobei die Ankerabschnitte (2, 3) Teil einer, insbesondere konischen, Hüllkontur (25) sind und dass durch Betätigen der Spreizmittel (5) ein, von den Ankerabschnitten (2, 3) aufgespannter Spreizwinkel, insbesondere ein Konuswinkel, der Hüllkontur (25) in Stufen oder stufenlos einstellbar ist, und wobei die Spreizmittel (5) einen Spindeltrieb umfassen, der eine drehbar gelagerte, insbesondere, rückwärtig, mit einem Antrieb (10) versehene Spindel (6) umfasst, durch deren Verdrehen ein Spreizreiter (8) entlang der Längsachse (L) der Spindel (6) zum Aufspreizen der Ankerabschnitte (2,3) verstellbar ist, wobei die Spindel (6) formschlüssig axial gesichert ist,
**dadurch gekennzeichnet,**
**dass** die Spindel (6) mit einem verdickten Abschnitt in einer zumindest abschnittsweise von einem Basisabschnitt (4) gebildeten Kavität (16) verdrehbar aufgenommen und die Spindel unverlierbar am Subtalar-Implantat (1) gehalten ist, und dass ein vorderer Rand des Basisabschnitts (4) umgebördelt ist und somit die Spindel fixiert.

2. Subtalar-Implantat nach Anspruch 1,
**dadurch gekennzeichnet,**
**dass** der Antrieb der Spindel (6) auf einer Axialseite des Implantates angeordnet ist, an der die Ankerabschnitte (2, 3) im gespreizten Zustand maximal beabstandet sind.

3. Subtalar-Implantat nach einem der Ansprüche 1 oder 2,
**dadurch gekennzeichnet,**
**dass** der Spreizreiter (8) unverlierbar auf der Spindel (6) gesichert ist.

4. Subtalar-Implantat nach Anspruch 3,
**dadurch gekennzeichnet,**
**dass** zur axialen Sicherung des Spreizreiters (8) auf der Spindel zumindest einer der Ankerabschnitte (2, 3) den Spreizreiter (8) zur axialen Sicherung in radialer Richtung, bezogen auf die Axialerstreckung der Spindel (6), übergreift.

5. Subtalar-Implantat nach einem der vorhergehenden Ansprüche,
**dadurch gekennzeichnet,**
**dass** der Spreizreiter (8) eine rechteckige Querschnittskontur und bevorzugt eine quaderförmige Hüllkontur aufweist.

6. Subtalar-Implantat nach einem der vorhergehenden Ansprüche,
**dadurch gekennzeichnet,**
**dass** der Spreizreiter (8) einen Antrieb, insbesondere umfassend von seitlichen Aussparungen, aufweist, über den mittels eines Werkzeugs ein Drehmoment zum Entfernen des Implantates und/oder zum Eindrehen des Implantates aufbringbar ist und/oder über den der Spreizreiter (8) und die Ankerabschnitte (2, 3) während der Betätigung der Spindel (6) drehfest relativ zu dieser zu halten.

7. Subtalar-Implantat nach einem der vorhergehenden Ansprüche,
**dadurch gekennzeichnet,**
**dass** die Ankerabschnitte (2, 3) sich diametral bezogen auf die Längsachse (L) gegenüberliegend und/oder, vorzugsweise gleichmäßig, in Umfangsrichtung verteilt angeordnet sind.

8. Subtalar-Implantat nach einem der vorhergehenden Ansprüche,
**dadurch gekennzeichnet,**
**dass** das vordere und das hintere Ende (14) über einen Durchgangskanal (24) zur Aufnahme eines Kirschnerdrahtes verbunden sind.

9. Subtalar-Implantat nach einem der vorhergehenden Ansprüche,
**dadurch gekennzeichnet,**
**dass** die Ankerabschnitte (2, 3) zum Verankern in menschlichen Knochen eine Oberflächenstruktur (11) aufweisen, insbesondere eine, vorzugsweise gemeinsame, Außengewindestruktur und/oder eine mehrere in Richtung der Längserstreckung des Implantates hintereinander angeordnete Rillen umfallende Rillenstruktur.

10. Subtalar-Implantat nach einem der vorhergehenden Ansprüche,
**dadurch gekennzeichnet,**
**dass** die Ankerabschnitte (2, 3) zusammen mit dem Basisabschnitt (4) aus einem Materialstück, insbesondere Titanstück, hergestellt sind.

11. Subtalar-Implantat nach einem der vorhergehenden Ansprüche,
**dadurch gekennzeichnet,**
**dass** die Ankerabschnitte (2, 3) in radialer Richtung federnd ausgebildet und/oder angeordnet sind.

12. Subtalar-Implantat nach einem der vorhergehenden Ansprüche,
**dadurch gekennzeichnet,**
**dass** in zumindest einem der Ankerabschnitte (2, 3) mindestens eine Längsnut (12) als Rotationssicherung eingeformt ist.

13. Subtalar-Implantat nach einem der vorhergehenden Ansprüche,
**dadurch gekennzeichnet,**
**dass** ein maximaler Durchmesser (a1, a2) des Implantates um eine Strecke von mindestens 2mm veränderbar ist, wobei der Durchmesser aus einem Wertebereich zwischen 5mm und 20mm, vorzugsweise zwischen 10mm und 16mm gewählt ist.

14. System, umfassend ein Subtalar-Implantat nach einem der vorhergehenden Ansprüche und ein Werkzeug zum Betätigen der Spindel (6) und/oder zum Zusammenwirken mit einem Antrieb des Spreizreiters (8).

## Claims

1. A subtalar implant for stabilising the subtalar joint in the human foot, having a longitudinal axis (L) extending between a front end (13) and a rear end (14), having a first anchor section (2) arranged to the side of the longitudinal axis (L) and a second anchor section (3) arranged to the side of the longitudinal axis (L), wherein spreading means (5) are provided for spreading apart the implant (1) by relative displacement of the first and second anchor sections (2, 3); wherein the anchor sections (2, 3) are part of a sleeve contour (25), in particular of a conical sleeve contour; and wherein an angle of spread, in particular a cone angle, which is spanned by the anchor sections (2, 3) of the sleeve contour (25) can be set in steps or continuously by actuation of the spreading means (5); and wherein the spreading means (5) comprise a spindle drive which comprises a rotatably supported spindle (6), in particular provided with a drive (10), to the rear, with a spreading slide (8) being adjustable along the longitudinal axis (L) of the spindle (6) for spreading apart the anchor sections (2, 3) by rotation of said spindle, wherein the spindle (6) is secured axially with shape matching,
**characterised in that**
a thickened section of the spindle (6) is rotatably received in a cavity (16) formed at least in part sectionally by a base section (4) and the spindle is held non-losably at the subtalar implant (1), and **in that** a front margin of the base section (4) is beaded and thus fixes the spindle.

2. A subtalar implant in accordance with claim 1,
**characterised in that**
the drive of the spindle (6) is arranged on an axial side of the implant at which the anchor sections (2, 3) are at a maximum spacing from one another in the spread apart state.

3. A subtalar implant in accordance with claim 1 or claim 2,
**characterised in that**
the spreading slide (8) is secured in a non-losable manner on the spindle (6).

4. A subtalar implant in accordance with claim 3,
**characterised in that**
at least one of the anchor sections (2, 3) engages over the spreading slide (8) for the axial securing in the radial direction, with respect to the axial extent of the spindle (6), for the axial securing of the spreading slide (8) on the spindle.

5. A subtalar implant in accordance with any one of the preceding claims,
**characterised in that**
the spreading slide (8) has a rectangular cross-section and preferably has a parallelepiped sleeve contour.

6. A subtalar implant in accordance with any one of the preceding claims,
**characterised in that**
the spreading slide (8) has a drive, in particular comprising lateral cut-outs, via which a torque can be applied by means of a tool for removing the implant and/or for screwing in the implant and/or via which the spreading slide (8) and the anchor sections (2, 3) can be held rotationally fixedly relative to the spindle (6) during the actuation thereof.

7. A subtalar implant in accordance with any one of the preceding claims,
**characterised in that**
the anchor portions (2, 3) are diametrically opposed with respect to the longitudinal axis (L) and/or are arranged, preferably uniformly, distributed in the circumferential direction.

8. A subtalar implant in accordance with any one of the preceding claims,
**characterised in that**
the front end and the rear end (14) are connected via a throughgoing passage (24) for receiving a Kirschner wire.

9. A subtalar implant in accordance with any one of the preceding claims,
**characterised in that**
the anchor sections (2, 3) for anchoring in human bone have a surface structure (11), in particular an external thread structure, preferably a common external thread structure, and/or a groove structure comprising a plurality of grooves arranged in succession in the direction of the longitudinal extent of the implant.

10. A subtalar implant in accordance with any one of the preceding claims,
**characterised in that**
the anchor sections (2, 3) are produced together with a base portion (4) from a material piece, in particular a piece of titanium.

11. A subtalar implant in accordance with any one of the preceding claims,
**characterised in that**
the anchor sections (2, 3) are configured and/or arranged resiliently in the radial direction.

12. A subtalar implant in accordance with any one of the preceding claims,
**characterised in that**
at least one longitudinal groove (12) is formed as a security against rotation in at least one of the anchor sections (2, 3).

13. A subtalar implant in accordance with any one of the preceding claims,
**characterised in that**
a maximum diameter (a1, a2) of the implant can be changed by a distance of at least 2 mm, with the diameter being selected from a value range between 5 mm and 20 mm, preferably between 10 mm and 16 mm.

14. A system comprising a subtalar implant in accordance with any one of the preceding claims and a tool for actuating the spindle (6) and/or for cooperating with a drive of the spreading slide (8).

## Revendications

1. Implant sous-astragalien pour stabiliser l'articulation sous-astragalienne dans le pied humain, présentant un axe longitudinal (L) s'étendant entre une extrémité antérieure (13) et une extrémité postérieure (14), comprenant un premier tronçon d'ancrage (2) agencé latéralement par rapport à l'axe longitudinal (L) et un second tronçon d'ancrage (3) agencé latéralement par rapport à l'axe longitudinal (L), dans lequel sont prévus des moyens d'écartement (5) pour écarter l'implant (1) par déplacement relatif du premier et du second tronçon d'ancrage (2, 3), et dans lequel les tronçons d'ancrage (2, 3) font partie d'un contour enveloppe (25), en particulier conique, et dans lequel, par actionnement des moyens d'écartement (5) un angle d'écartement défini par les tronçons d'ancrage (2, 3), en particulier un angle conique, du contour enveloppe (25) est réglable par gradins ou en continu, et dans lequel les moyens d'écartement (5) incluent un entraînement à broche qui comprend une broche (6) montée en rotation et dotée d'un entraînement (10), en particulier du côté postérieur, de sorte que la rotation de la broche déplace un curseur d'écartement (8) le long de l'axe longitudinal (L) de la broche (6) pour écarter les tronçons d'ancrage (2, 3), la broche (6) étant bloquée axialement par coopération de formes,
**caractérisé en ce que**
la broche (6) est reçue avec faculté de rotation avec un tronçon épaissi dans une cavité (16) formé au moins localement par une portion de base (4) et la broche est retenue de manière imperdable sur l'implant sous-astragalien (1), et **en ce qu'**une bordure antérieure de la portion de base (4) est rabattue et fixe ainsi la broche.

2. Implant sous-astragalien selon la revendication 1,
**caractérisé en ce que** l'entraînement de la broche (6) est agencé sur un côté axial de l'implant auquel les tronçons d'ancrage (2, 3) sont éloignés au maximum dans la situation écartée.

3. Implant sous-astragalien selon l'une des revendications 1 ou 2,
**caractérisé en ce que** le curseur d'écartement (8) est bloqué de manière imperdable sur la broche (6).

4. Implant sous-astragalien selon la revendication 3,
**caractérisé en ce que**, pour le blocage axial du curseur d'écartement (8) sur la broche, au moins un des tronçons d'ancrage (2, 3) coiffe le curseur d'écartement (8) pour le blocage axial en direction radiale, par référence à l'extension axiale de la broche (6).

5. Implant sous-astragalien selon l'une des revendications précédentes,
**caractérisé en ce que** le curseur d'écartement (8) présente un contour de section transversale rectangulaire et de préférence un contour enveloppe en forme de parallélépipède.

6. Implant sous-astragalien selon l'une des revendications précédentes,
**caractérisé en ce que** le curseur d'écartement (8) comprend un entraînement, incluant en particulier des évidements latéraux, via lequel un couple de rotation peut être appliqué au moyen d'un outil pour enlever l'implant et/ou pour enfoncer l'implant, et/ou via lequel le curseur d'écartement (8) et les tronçons d'ancrage (2, 3) sont retenus solidairement en rotation par rapport à la broche (6) pendant l'actionnement de celle-ci.

7. Implant sous-astragalien selon l'une des revendications précédentes,
**caractérisé en ce que** les tronçons d'ancrage (2, 3) sont diamétralement opposés par rapport à l'axe longitudinal (L) et/ou sont agencés de façon répartie en direction périphérique, de préférence de manière régulière.

8. Implant sous-astragalien selon l'une des revendications précédentes,
**caractérisé en ce que** l'extrémité antérieure et l'extrémité postérieure (14) sont reliées via un canal traversant (24) pour la réception d'un fil de Kirschner.

9. Implant sous-astragalien selon l'une des revendications précédentes,
**caractérisé en ce que** les tronçons d'ancrage (2, 3) comportent, pour l'ancrage dans des os humains, une structure de surface (11), en particulier une structure en pas de vis extérieur, de préférence commune, et/ou une structure à rainure comportant plusieurs rainures agencées les unes derrière les autres en direction de l'extension longitudinale de l'implant.

10. Implant sous-astragalien selon l'une des revendications précédentes,
**caractérisé en ce que** les tronçons d'ancrage (2, 3) sont réalisés, conjointement avec la portion de base (4), en un morceau de matériau, en particulier un morceau de titane.

11. Implant sous-astragalien selon l'une des revendications précédentes,
**caractérisé en ce que** les tronçons d'ancrage (2, 3) sont réalisés et/ou agencés avec effet ressort en direction radiale.

12. Implant sous-astragalien selon l'une des revendications précédentes,
**caractérisé en ce qu'**au moins une gorge longitudinale (12) est ménagée dans l'un au moins des tronçons d'ancrage (2, 3) à titre de blocage antirotation.

13. Implant sous-astragalien selon l'une des revendications précédentes,
**caractérisé en ce qu'**un diamètre maximum (a1, a2) de l'implant est variable d'une amplitude d'au moins 2 mm, et le diamètre est choisi dans une plage de valeurs entre 5 mm et 20 mm, de préférence entre 10 mm et 16 mm.

14. Système comprenant un implant sous-astragalien selon l'une des revendications précédentes et un outil pour actionner la broche (6) et/ou pour coopérer avec un entraînement du curseur d'écartement (8).
